(19) Europäisches Patentamt
European Patent Office
Office européen des brevets

(11) **EP 3 960 219 A1**

(12) **EUROPEAN PATENT APPLICATION**

(43) Date of publication:
**02.03.2022 Bulletin 2022/09**

(21) Application number: **21193526.7**

(22) Date of filing: **27.08.2021**

(51) International Patent Classification (IPC):
**A61M 5/14** *(2006.01)*    **A61M 5/142** *(2006.01)*
**A61M 5/172** *(2006.01)*    **G16H 20/17** *(2018.01)*

(52) Cooperative Patent Classification (CPC):
**A61M 5/14; A61M 5/14248; A61M 5/1723;
G16H 20/17;** A61M 2205/3569

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2020 US 202017008054**

(71) Applicant: **Insulet Corporation
Acton MA 01720 (US)**

(72) Inventors:
• **ZADE, Ashutosh
San Diego (US)**
• **LEE, Joon Bok
Acton (US)**
• **ZHENG, Yibin
Hartland (US)**

(74) Representative: **Peterreins Schley
Patent- und Rechtsanwälte PartG mbB
Hermann-Sack-Strasse 3
80331 München (DE)**

(54) **TECHNIQUES FOR DETERMINING INSULIN FORMULATIONS IN AN AUTOMATED INSULIN DELIVERY SYSTEM**

(57) Techniques and devices for determining and generating insulin formulations are described. An automated insulin delivery device (100) may include a wireless interface with a glucose sensor (106) for providing blood glucose concentration readings of a user, a plurality of insulin reservoirs (112), each of the plurality of insulin reservoirs holding a different one of a plurality of insulin types, each of the plurality of insulin types having a different strength, a storage medium (118) storing programming instructions and user glucose information, the user glucose information comprising the blood glucose concentration readings, predicted future blood glucose concentration readings, and user activity information, determining an insulin strength based on the user glucose information, determining an insulin formulation ratio to generate an insulin formulation having the insulin strength using the plurality of insulin types, an insulin formulation device to generate the insulin formulation; and an insulin delivery component (102) to deliver the insulin formulation to the user.

FIG. 1

## Description

## BACKGROUND

Automated Insulin Delivery (AID) or Automatic Glucose Control (AGC) systems operate to reduce the burden of Type-1 diabetes by providing automated insulin dosage control. AID systems need continuously monitored glucose data (CGM) to run a constant feedback control algorithm to determine modifications needed to the insulin dose in response to CGM data. In addition, AID systems require users to announce meals, which allows an automated AID process to start releasing insulin to counterbalance an imminent rise in blood glucose due to meal ingestion. Such processes are generally referred as postprandial control and are particularly challenging due to mismatches between glycemic response to certain foods, such as carbohydrates, and comparatively slower onset and peak action of subcutaneously delivered insulin. Furthermore, the postprandial response has additional challenges as each meal type (for instance, high-carb, high-fat, and high-fiber) may have different individual characteristics that may materially affect patient glucose levels. Accordingly, conventional AID systems are not capable of providing an effective and accurate postprandial response for patients.

## BRIEF DESCRIPTION OF THE DRAWINGS

FIG. 1 depicts an environment including an AID system suitable for practicing an exemplary embodiment.
FIG. 2 depicts a block diagram of a device suitable for performing methods of exemplary embodiments described herein.
FIG. 3 depicts a first logic flow according to some embodiments.
FIG. 4 depicts a first second flow according to some embodiments.
FIG. 5 depicts a third logic flow according to some embodiments.
FIG. 6 depicts a fourth logic flow according to some embodiments.
FIG. 7 depicts an illustrative plot of an approximation of a meal event according to some embodiments.
FIG. 8 depicts an illustrative plot of a peak time window to coincide insulin action to peak glucose response according to some embodiments.
FIG. 9 depicts an illustrative plot comparing long lasting insulin versus rapid-acting insulin according to some embodiments.

The drawings are not necessarily to scale. The drawings are merely representations, not intended to portray specific parameters of the disclosure. The drawings are intended to depict exemplary embodiments of the disclosure, and therefore should not be considered as limiting in scope. In the drawings, like numbering represents like elements.
Furthermore, certain elements in some of the figures may be omitted, or illustrated not-to-scale, for illustrative clarity. The cross-sectional views may be in the form of "slices," or "near-sighted" cross-sectional views, omitting certain background lines otherwise visible in a "true" cross-sectional view, for illustrative clarity. Furthermore, for clarity, some reference numbers may be omitted in certain drawings.

## DETAILED DESCRIPTION

The described technology generally includes an insulin formulation process operative to generate insulin formulations for controlling patient glucose levels based on event information. In some embodiments, insulin formulation processes may operate to control insulin formulation for Automated Insulin Delivery (AID), Automatic Glucose Control (AGC), or other automated systems to derive correct insulin strength requirements based upon current needs for correcting blood glucose.
In some embodiments, an AID device may include or otherwise have access to a plurality of insulin types. The plurality of insulin types may have different strengths. Non-limiting examples of insulin types may include rapid- or fast-acting insulin, long-acting or basal insulin, short-acting insulin, intermediate acting insulin (NPH), and/or the like. Each insulin type may have a strength or other characteristic. For example, rapid-acting insulin is usually taken just after or before meals for controlling blood sugar spikes, often begins to work in 10 to 15 minutes, peaks in about 1 hour, and lasts for about 3 to 5 hours. For rapid-acting insulin, however, the dose affects the duration of action; for example, small units can last 3 hours or less, while 20 or 25 units can last up to 5 hours. The rapid-acting insulin may mimic meal-stimulated insulin secretion. Rapid-acting insulin analogs may improve the ability to match insulin dose to carbohydrate intake and ensure that the insulin and glucose reach the blood at approximately the same time.
In contrast, long-acting insulin gives a slow and steady release of insulin that helps control blood sugar between meals and/or overnight. The duration of long-acting insulin action is generally longer than rapid-acting insulin. Long-acting insulin exists in different formulations such as U100 (100 units/mL), U200 and U500. While U500 is generally reserved for patients with extreme insulin resistance, the insulin formulation process may operate to formulate actual doses personalized by using the correct concentration of the long acting insulin.

In one embodiment, for example, an AID device may have two insulin reservoirs and the insulin formulation process may operate to determine an insulin formulation, for example, that may mix or otherwise combine the rapid-acting insulin and long-acting insulin to generate a specific insulin formulation having a specific strength. The insulin formulation may be delivered for a specific purpose or event, such as after meal detection. The exact ratio of the different insulin types may not be fixed; rather, the insulin formulation process may be calculated based upon various event factors.

For example, in some embodiments, the insulin formulation process may monitor user glucose readings (for instance, continuous glucose monitoring (CGM) data) and, with AID feedback control, determine insights regarding one or more events that may affect glucose levels. In various embodiments, the insulin formulation process may determine an event value for one or more events. Non-limiting examples of events may include a meal event (e.g., a start of a meal), a carbohydrate ingestion event (e.g., between-meal snacks, "rescue" carbohydrates, and/or the like), and/or an elevated activity event (e.g., exercise or increased activity). An event value may indicate that an event has occurred (for example, a meal event value may be zero or below a meal event threshold if no meal event is detected and a non-zero value or above a threshold value if a meal event has been detected) and other information, such as the confidence in the event, the intensity of the event, and/or the like. The insulin formulation process may determine an insulin strength or other formulation factor based on the event values. In various embodiments, the insulin formulation process may cause an insulin formulation to be generated by combining different insulin types to achieve the insulin formulation. In addition, the insulin formulation process may determine when, if, or how (for instance, delivery rate) to deliver the insulin to the user based on the event values.

In some embodiments, events monitored by the insulin formulation process may include a meal event, a carbohydrate ingestion event, and/or an increased activity event. Although a meal event, a carbohydrate ingestion event, and/or an increased activity event may be used in examples in the present disclosure, embodiments are not so limited as any type of event that may be monitored is contemplated herein. In one embodiment, for example, a meal event may be determined by predicting a CGM trend within a trend duration based upon recent past CGM readings and/or an amount of suggested insulin (for instance, a current insulin dosage or dosage recommendation). In various embodiments, deviation of a current/past CGM trend from the predicted CGM trend may indicate a meal event, alone or coupled with various secondary factors, such as time of day, historical mealtimes (either learned or entered by the user), user location, historical CGM trends, and/or the like.

In another embodiment, for example, a carbohydrate ingestion for rescue of hypoglycemia or between-meal snacks may be similarly deduced by secondary factors (such as time of day, hours from previous meals) alone or secondary factors in combination with a difference between predicted versus actual CGM trends. In a further embodiment, for example, an elevated activity may be detected based on user input, an activity sensor (for instance, an internet-of-things (IoT) device, fitness tracker, step tracker, pulse oximeter, accelerometer, GPS information, and the like), enabling of device hypoglycemia mode, increased insulin sensitivity, and the like. A determination of an increased activity event may be achieved via finding a threshold difference between predicted CGM trend and actual CGM trend. In addition, certain activities may be detected based upon learned activity times from user history information (for instance, the user exercises at 7:00 am, the user has never exercised after 6:00 pm, and the like), user location (for instance, GPS information indicates user is at a gym or jogging path), and or user input. In some embodiments, increased activity, which may lead to increased insulin absorption, may indicate that an insulin formulation with less insulin (or a lower or zero amount of rapid acting insulin) may be needed. In addition, increased activity may be an indicator of an increased risk of hypoglycemia. Accordingly, in some embodiments, an insulin formulation process may limit the amount of insulin and/or suspend insulin delivery based, at least in part, on detected activity.

Each of the detected events, as well as characteristics thereof, may require different types and/or strengths of insulin. The insulin formulation process may determine the actual strength needed to correct the CGM based upon the identified event, formulate the insulin from available insulin reservoirs, and/or recommend a combined insulin quantity to quickly bring blood glucose in control.

Some embodiments may provide multiple technological advantages over conventional systems, including improvements in computing technology and practical applications of the described methods. For example, insulin formulation processes or methods according to some embodiments may be applied to the application of determining an insulin formulation for a user to maintain a healthy glucose level, for example, based on events being experienced by the user. In another example, insulin formulation processes or methods may be applied to making an insulin formulation via components (e.g., pumps, reservoirs, and the like) of an insulin delivery device. In a further example, insulin formulation processes or methods may be applied to delivering a specifically formulated insulin dosage determined particularly for a user based on events being experienced by the user.

Accordingly, some embodiments may provide multiple technological advantages over existing systems. In one non-limiting technological advantage, some embodiments may provide for an AID device having a plurality of insulin types that may be delivered to a user using a single device. In another non-limiting technological advantage, some embodiments may provide processes for determining an insulin formulation, including a specific insulin strength based on a combination of different types of insulin compounds, based on events being experienced by the user. Conventional computing systems are able to determine CGM information and generate an insulin dosage for a single type of insulin. Therefore, some embodiments may provide improvements in computing technology that overcome the limits of existing systems by providing a computing device or system (including an AID device) capable of determining events effecting recommended insulin dosages and effectuating the insulin dosages by generating insulin dosages formulated by mixing different types of insulin. Other advantages and applications are described and/or would be evident to those of ordinary skill in the art based on the present disclosure.

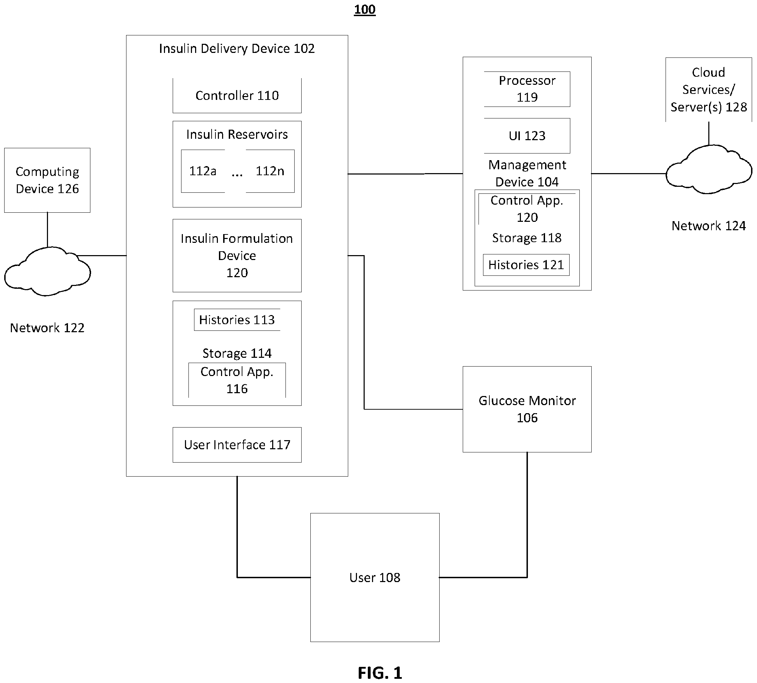

**FIG. 1** depicts an illustrative drug delivery system 100 that is suitable for delivering insulin to a user 108 in an exemplary embodiment. The drug delivery system 100 includes an insulin delivery device 102. The insulin delivery device 102 may be a wearable device that is worn on the body of the user 108. The insulin delivery device 102 may be directly coupled to a user, e.g., directly attached to a body part and/or skin of the user 108 via an adhesive or the like. In an example, a surface of the insulin delivery device 102 may include an adhesive to facilitate attachment to the user 108.

The insulin delivery device 102 may include a controller 110. The controller 110 may be implemented in hardware, software, or any combination thereof. The controller 110 may, for example, be a processor, microprocessor, a logic circuit, a field programmable gate array (FPGA), an application specific integrated circuit (ASIC) or a microcontroller coupled to a memory. The controller 110 may maintain a date and time as well as perform other functions, e.g., perform calculations and the like. The controller 110 may be operable to execute a control application 116 stored in the storage 114 that enables the controller 110 to direct operation of the insulin delivery device 102. The storage 114 may hold histories 113 for a user, such as a history of automated insulin deliveries, a history of bolus insulin deliveries, meal event history, exercise event history, location history, and the like. In addition, the controller 110 may be operable to receive data or information. The storage 114 may include both primary memory and secondary memory. The storage may include random access memory RAM, read only memory ROM, optical storage, magnetic storage, removable storage media, solid state storage or the like.

The insulin delivery device 102 may include a plurality of insulin reservoirs 112a-n for storing insulin for delivery to the user 108 as warranted. In some embodiments, the insulin reservoirs 112a-n may each hold a different type of insulin, such as rapid-acting, slow-acting, long-acting, basal, intermediate acting, and the like. In some embodiments, an insulin formulation device 120 may be used to generate insulin formulations using insulin stored in insulin reservoirs 112a-n. In various embodiments, insulin formulation device 120 may be configured to measure, mix, combine, or otherwise process insulin arranged in insulin reservoirs 112a-n to generate insulin formulations according to some embodiments. In some embodiments, at least one of insulin reservoirs 112a-n may include at least one formulation reservoir containing a current insulin formulation generated according to some embodiments.

A fluid path to the user 108 may be provided, and the insulin delivery device 102 may expel the insulin from insulation formulation device 120 and/or one of insulin reservoirs 112a-n to deliver the insulin to the user 108 via the fluid path. The fluid path may, for example, include tubing coupling the drug delivery device 102 to the user 108, e.g., tubing coupling a cannula to the insulin reservoir 112.

There may be one or more communications links with one or more devices physically separated from the insulin delivery device 102 including, for example, a management device 104 of the user and/or a caregiver of the user and/or a glucose monitor 106. The communication links may include any wired or wireless communication link operating according to any known communications protocol or standard, such as Bluetooth®, Wi-Fi, a near-field communication standard, a cellular standard, or any other wireless protocol The insulin delivery device 102 may also include a user interface 117, such as an integrated display device for displaying information to the user 108 and in some embodiments, receiving information from the user 108. The user interface 117 may include a touchscreen and/or one or more input devices, such as buttons, a knob or a keyboard.

The insulin delivery device 102 may interface with a network 122. The network 122 may include a local area network (LAN), a wide area network (WAN) or a combination thereof. A computing device 126 may be interfaced with the network, and the computing device may communicate with the insulin delivery device 102.

The drug delivery system 100 may include a glucose monitor 106 for sensing the blood glucose concentration levels of the user 108. The glucose monitor 106 may provide periodic blood glucose concentration measurements and may be a continuous glucose monitor CGM, or another type of device or sensor that provides blood glucose measurements. The glucose monitor 106 may be physically separate from the insulin delivery device 102 or may be an integrated component thereof. The glucose monitor 106 may provide the controller 110 with data indicative of measured or detected blood glucose levels of the user 108. The glucose monitor 106 may be coupled to the user 108 by, for example, adhesive or the like and may provide information or data on one or more medical conditions and/or physical attributes of the user 108. The information or data provided by the glucose monitor 106 may be used to adjust drug delivery operations of the insulin delivery device 102.

The drug delivery system 100 may also include the management device 104. The management device 104 may be a special purpose device, such as a dedicated personal diabetes manager PDM device. The management device 104 may be a programmed general purpose device, such as any portable electronic device including, for example, a dedicated controller, such as processor, a smartphone, or a tablet. The management device 104 may be used to program or adjust operation of the drug delivery device 102 and/or the sensor 104. The management device 104 may be any portable electronic device including, for example, a dedicated controller, a smartphone, or a tablet. In the depicted example, the management device 104 may include a processor 119 and a storage 118. The processor 119 may execute processes to manage a user's blood glucose levels and for control the delivery of the drug or therapeutic agent to the user 108. The processor 119 may also be operable to execute programming code stored in the storage 118. For example, the storage may be operable to store one or more control applications 120 for execution by the processor 119. The storage 118 may store the control application 120, histories 121 like those described above for the insulin delivery device 102 and other data and/or programs.

The management device 104 may include a user interface 123 for communicating with the user 108. The user interface may include a display, such as a touchscreen, for displaying information. The touchscreen may also be used to receive input when it is a touch screen. The user interface 123 may also include input elements, such as a keyboard, button, knobs or the like.

The management device 104 may interface with a network 124, such as a LAN or WAN or combination of such networks. The management device 104 may communicate over network 124 with one or more servers or cloud services 128. The role that the one or more servers or cloud services 128 may play in the exemplary embodiments will be described in more detail below.

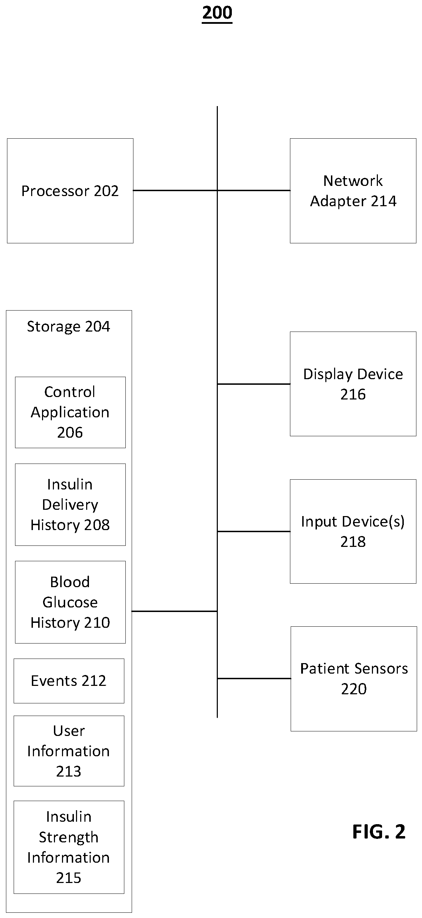

**FIG. 2** depicts a block diagram of a device 200 suitable for performing the methods that will be described in more detail below. The device 200 may, in different exemplary embodiments, be the insulin delivery device 102, the management device 104, the computing device 126, or the one or more servers 128. Where the device is the computing device 126, or the one more servers or cloud services 128, the device 200 may act in cooperation with the management device 104 and the insulin delivery device 102 to perform the methods. The device 200 includes a processor 202 for executing programming instructions. The processor 202 has access to a storage 204. The storage 204 may store an application 206 for performing the methods. This application 206 may be executed by the processor 202. The storage 204 may store an insulin delivery history 208 for the user. The insulin delivery history 208 may contain data regarding the amount of insulin delivered as well as the date and time of the deliveries. The insulin delivery history 208 may also identify if each delivery is a basal delivery or a bolus delivery. In some embodiments, insulin delivery history 208 may include a particular formulation of insulin delivered. The storage 204 may store the blood glucose history 210. The blood glucose history 210 may include blood glucose concentration readings as well as the date and time of such readings. These values may be obtained by the glucose monitor 106. The storage 204 additionally may store information regarding events 212, like meal events, exercise events, and the like. In some embodiments, the storage 204 may store user information 213 associated with the user, such as temperature, heartrate, number of steps, location, and the like that may be measured via patient sensors 220. The patient sensors 220 may include sensors operative to determine or measure information associated with a user, such as IoT devices, fitness trackers, heart rate monitors, pulse oximeters, accelerometers, GPS devices, and the like.

The device 200 may include a network adapter 214 for interfacing with networks, like networks 122 and 124. The device 200 may have a display device 216 for displaying video information. The display device 216 may be, for instance, a liquid crystal display LCD device, a light emitting diode LED device, etc. The device 200 may include one or more input devices 218 for enabling input to be received. Examples of input devices may include keyboards, mice, pointing devices, touchscreen displays, button, knobs or the like.

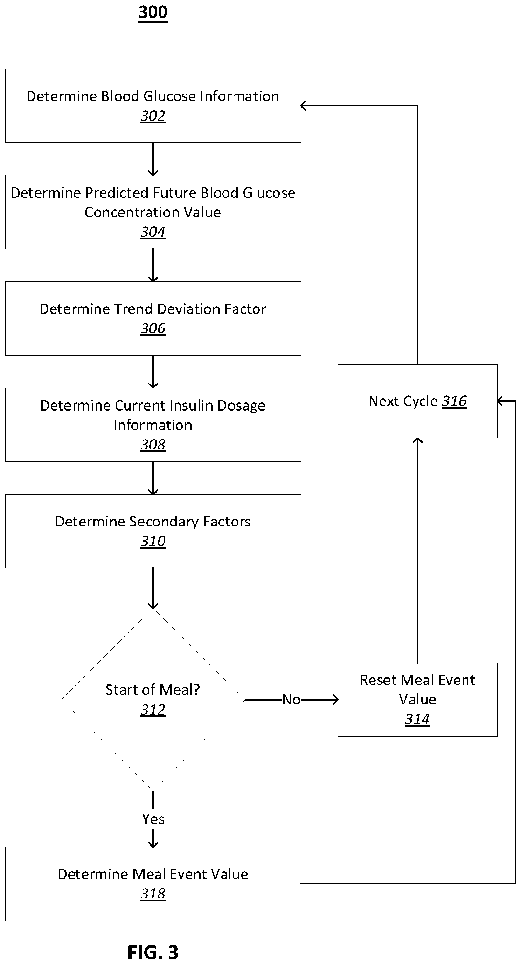

The device 200 such as the insulin delivery device 102 may perform the steps depicted in the flowcharts 300-600 of FIGS 3-6, for example, to determine and/or value events, determine insulin formulations according to some embodiments, and/or to set other insulin delivery settings for the user. For purposes of the discussion below it will be assumed that the device 200 is the insulin delivery device 102. The insulin delivery settings may include, but are not limited to, what dosage of insulin to deliver to a user and when to deliver the insulin to the user. The dosage amount may be zero in instances where it is determined that insulin delivery is not required or needs to be suspended.

Included herein are one or more logic flows representative of exemplary methodologies for performing novel aspects of the disclosed architecture. While, for purposes of simplicity of explanation, the one or more methodologies shown herein are shown and described as a series of acts, those skilled in the art will understand and appreciate that the methodologies are not limited by the order of acts. Some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all acts illustrated in a methodology may be required for a novel implementation.

A logic flow may be implemented in software, firmware, hardware, or any combination thereof. In software and firmware embodiments, a logic flow may be implemented by computer executable instructions stored on a non-transitory computer readable medium or machine readable medium. The embodiments are not limited in this context.

**FIG. 3** illustrates an embodiment of a logic flow 300. Logic flow 300 may be representative of some or all of the operations executed by one or more embodiments described herein, such as insulin delivery device 102, computing device 126, cloud services/servers 128, management device 104, glucose monitor 106, and/or device 200 . In some embodiments, logic flow 300 may be representative of some or all of the operations of an insulin formulation process according to some embodiments, for example, to determine a meal event value.

At block 302, logic flow 300 may determine blood glucose information at block 302. For example, an insulin formulation process may determine current and/or historical blood glucose information for a user. Logic flow 300 may determine predicted future blood glucose concentration values at block 302. For example, one suitable way for determining the predicted future blood glucose concentration value in block 304 may be expressed by the following

Equation (1):

$$G_p(k+1) = b_0 G_{new}(k) + b_1 G_{new}(k-1) + \cdots b_n G_{new}(k-n) + I(k-1) + I(k-2)$$
$$+ \cdots I(k-n)$$

where $G_p(k+1)$ is the predicted future blood glucose concentration value at control cycle $k$, $G_{new}(k)$ is the blood glucose concentration reading for control cycle $k$, $b_i$ is a weighting coefficient for the ith control cycle before the current control cycle and $I(k)$ is the insulin action for insulin delivered during the kth control cycle. Embodiments are not limited in this context as other processes for determining predicted blood glucose values may be used.

At block 306, logic flow 300 may determine a trend deviation factor. For example, the insulin formulation process may operate to compare the blood glucose information (which may include current and historical blood glucose concentrations) to predicted future blood glucose values to determine if there is a deviation. In some embodiments, a deviation threshold may be used to determine if there has been a deviation substantive enough to trigger a determination that there is a deviation between the blood glucose information and the predicted future blood glucose values (i.e., does the current trend appear as though it will deviate from the expected, predicted trend over a threshold amount). At block 308, logic flow 300 may determine current insulin dosage information. Non-limiting examples of current insulin dosage information may include insulin units, delivery rate, insulin type, insulin formulation/strength, and the like. Logic flow 300 may determine secondary factors at block 310. In some embodiments, secondary factors may include other factors associated with the user that may be used to determine whether a meal has started, including, without limitation, time of day, historical meal times (either determined via an insulin formulation process or entered by a user), location information (e.g., GPS or other data indicates the user is in a restaurant), external device data (e.g., a connected or "smart" appliance indicates that the user is cooking), image-based information (e.g., AI or ML techniques for evaluating images of GGM graphs), glucose trajectories, and the like.

In addition, for a meal event, secondary considerations may include the type of meal (e.g., if the user indication or predictions suggests a high fat meal, the final formulation may contain more long acting insulin, or onset of rapid acting type can be delayed), duration of the meal (e.g., if meal is extended, the food intake spreads over longer duration (e.g., special meals, events, holiday parties, etc.) and multiple formulations may be necessary), size of the meal (e.g., the indicated size of the meal is helpful to assess how much, and the proportions of, the insulin may be needed), and the like.

At block 312, logic flow 300 may determine whether a meal has started (or a value associated with a current meal event) based on the determinations of blocks 306, 308, and/or 310. If a meal has not started, logic flow 300 may reset a meal event value at block 314 and restart the process for a next cycle at block 316. If it is determined that a meal has started, logic flow may determine a meal event value at block 318.

In some embodiments, event values, such as meal event value 318 may provide an indication of whether an event has occurred and/or an intensity of the event. For example, an event value may be a binary value (such as 0 for active and 1 for inactive). In another example, an event value may be on a scale (for instance, 1-100) indicating an intensity of the event (for instance, a meal event value over 10 indicates a meal event is active; a value over 20 indicates a small (or low carbohydrate) meal; a value over 50 indicates a large meal; and so on). Embodiments are not limited in this context.

In various embodiments, logic flow 300 may determine an active/inactive state of, and/or set a value for, a meal event by predicting a CGM trend within a trend duration based upon recent past CGM readings and/or an amount of suggested insulin (for instance, a current insulin dosage or dosage recommendation). In various embodiments, deviation of a current/past CGM trend from the predicted CGM trend may indicate a meal event, alone or coupled with various secondary factors, such as time of day, historical mealtimes (either learned or entered by the user), user location, historical CGM trends, and the like.

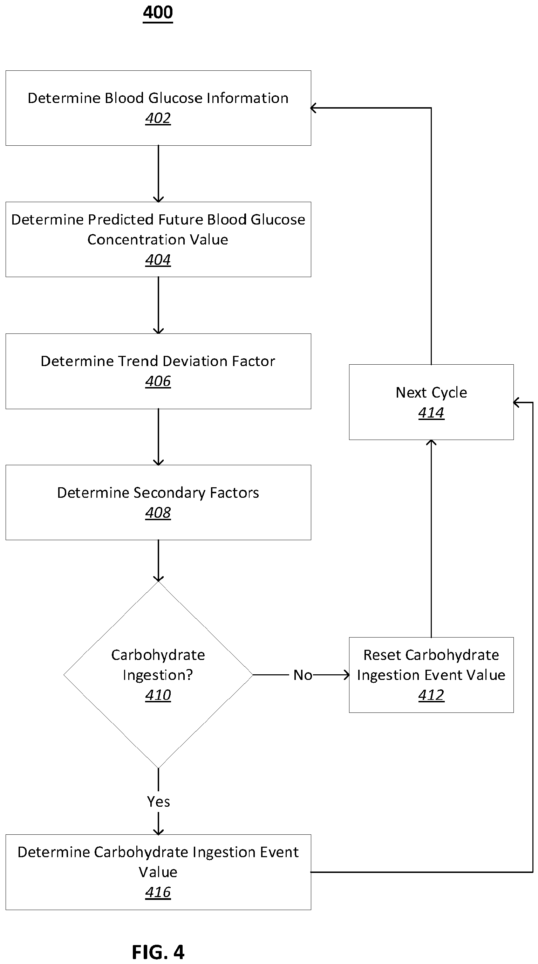

**FIG. 4** illustrates an embodiment of a logic flow 400. Logic flow 400 may be representative of some or all of the operations executed by one or more embodiments described herein, such as insulin delivery device 102, computing device 126, cloud services/servers 128, management device 104, glucose monitor 106, and/or device 200 . In some embodiments, logic flow 400 may be representative of some or all of the operations of an insulin formulation process according to some embodiments, for example, to determine a carbohydrate event value.

At block 402, logic flow 400 may determine blood glucose information. For example, an insulin formulation process may determine current and/or historical blood glucose information for a user. Logic flow 400 may determine predicted future blood glucose concentration values at block 402. For example, logic flow may determine the predicted future blood glucose concentration value in block 404 via Equation (1).

At block 406, logic flow 400 may determine a trend deviation factor. Logic flow 400 may determine secondary factors at block 408. At block 410, logic flow 400 may determine whether a carbohydrate ingestion event has started (or a value associated with a current carbohydrate ingestion event) based on the determinations of blocks 406 and/or 408. If a carbohydrate ingestion event has not started, logic flow 400 may reset a carbohydrate ingestion event value at block 412 and restart the process for a next cycle at block 414. If it is determined that a carbohydrate ingestion event has started, logic flow 400 may determine a carbohydrate ingestion event value at block 416.

In some embodiments, for example, logic flow 400 may operate to determine a carbohydrate ingestion event, such as carbohydrate ingestion for rescue of hypoglycemia or between-meal snacks, deduced based on secondary factors (such as time of day, hours from previous meals) alone or secondary factors in combination with a difference between predicted versus actual CGM trends.

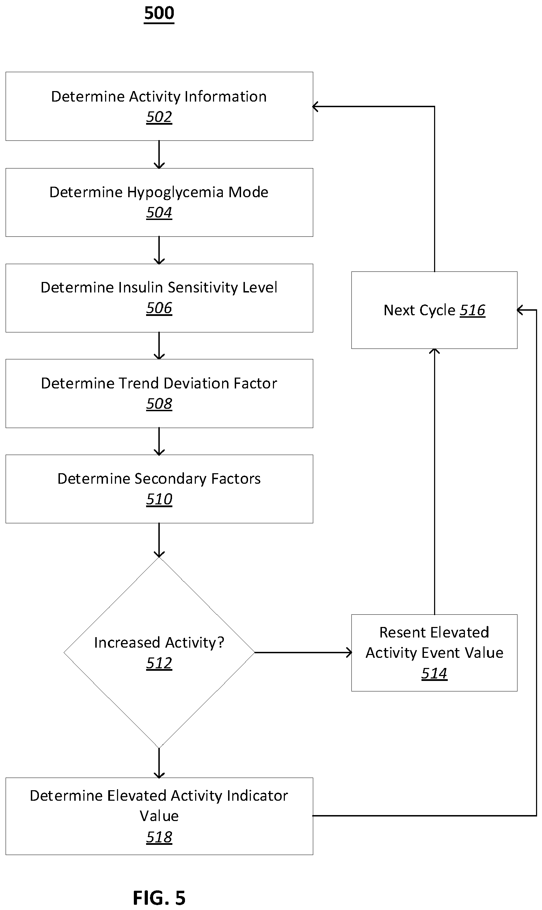

**FIG. 5** illustrates an embodiment of a logic flow 500. Logic flow 500 may be representative of some or all of the operations executed by one or more embodiments described herein, such as insulin delivery device 102, computing device 126, cloud services/servers 128, management device 104, glucose monitor 106, and/or device 200 . In some embodiments, logic flow 500 may be representative of some or all of the operations of an insulin formulation process according to some embodiments, for example, to determine an increased activity event value.

At block 502, logic flow 500 may determine activity information. For example, one or more sensors or devices may be configured to determine activity information associated with a user. Non-limiting examples of activity information may include information indicating that a user is at an elevated activity level, such as exercising, running, hiking, climbing stairs, and the like (or, conversely, that the user is inactive, such as lack of movement, or an indication that the user is driving, and the like). In another embodiment, an activity level may be determined based on direct user input.

Logic flow 500 may determine a hypoglycemia mode at block 504. For example, an AID or other device may indicate that the user is in a hypoglycemic state, which may be an indicator of increased activity (or, at least that this event state/value should incline toward a lower insulin strength (or even no insulin)). At block 506, logic flow 500 may determine an insulin sensitivity level of the user. Non-limiting examples of determining an insulin sensitivity may include using a various rules/calculations, such as the "1500" rule, or determining an insulin sensitivity factor.

At block 508, logic flow 500 may determine a trend deviation factor. Logic flow 500 may determine secondary factors at block 510. At block 512, logic flow 500 may determine whether an increased activity event has started (or a value associated with a current increased activity event) based on the determinations of blocks 502-510. If an increased activity event has not started, logic flow 500 may reset an increased activity event value at block 514 and restart the process for a next cycle at block 516. If it is determined that an increased activity event has started, logic flow 500 may determine an increased activity event value at block 518.

In some embodiments, for example, logic flow may determine an elevated activity based on user input, activity information (for instance, IoT device, fitness tracker, step tracker, pulse oximeter, accelerometer, GPS information, and the like), enabling of device hypoglycemia mode, increased insulin sensitivity, a trend deviation factor, and the like.

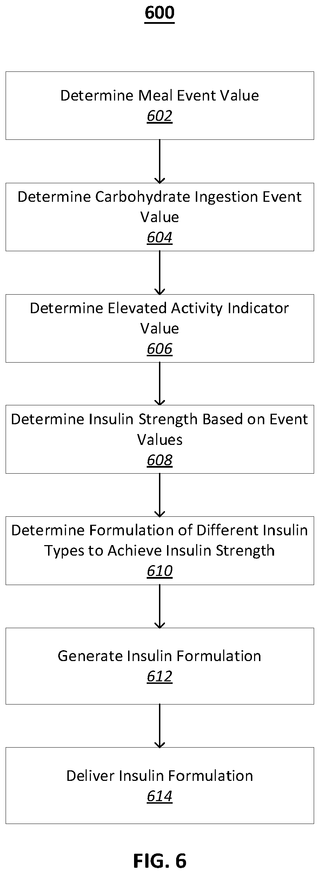

**FIG. 6** illustrates an embodiment of a logic flow 600. Logic flow 600 may be representative of some or all of the operations executed by one or more embodiments described herein, such as insulin delivery device 102, computing device 126, cloud services/servers 128, management device 104, glucose monitor 106, and/or device 200 . In some embodiments, logic flow 600 may be representative of some or all of the operations of an insulin formulation process according to some embodiments, for example, to determine and generate an insulin formulation.

At block 602, logic flow 600 may determine a meal event value, for instance, according to logic flow 300. At block 604, logic flow 600 may determine a carbohydrate ingestion event value, for instance, according to logic flow 400. At block 606, logic flow 600 may determine an elevated activity event value, for instance, according to logic flow 500.

Logic flow 600 may determine an insulin strength at block 606 based on the event values of blocks 602-606. For example, the insulin formulation process may access a lookup table, database, conversion information, or other data structure to convert the event values into an insulin strength (or directly into an insulin formulation). In various embodiments, storage 204 may store insulin strength information 215 to convert event values into an insulin strength (or directly into an insulin formulation). For example, insulin formulation process may add up the values and look up the compound value in insulin strength information 215 to determine an insulin strength (or directly into an insulin formulation). For instance, if all three events are active, then an insulin strength may be X; if only one is active, the insulin strength may be Y; and so on. In another example, if an event score (or the aggregate event score) is greater than a first threshold, then the insulin strength is X (or a first formulation is selected); if the event score is greater than a second threshold, the insulin strength is Y (or a second formulation is selected); and so on. In some embodiments, the events (and their respective event values) may be weighted. For instance, for a first user, a meal event may be weighted more because it has a larger effect on his glucose concentration, while for a second user, an elevated activity event may be weighted more because it has a larger effect on her glucose concentration than the other events. Embodiments are not limited in this context as any method for correlating event values (or active events) with insulin strengths and/or formulations is contemplated herein.

At block 610, logic flow 600 may determine an insulin formulation based on the insulin strength. For example, the insulin formulation process may determine the amounts of different types of insulin to mix to generate a desired strength. In some embodiments, an insulin formulation may include one type of insulin. In other embodiments, an insulin formulation may include a plurality of different types of insulin, such as two types, three types, or four types. In some embodiments, the different types of insulin may be mixed together in different ratios to generate the insulin formulations. Logic flow 600 may generate the insulin formulation at block 612. For example, insulation formulation device 120 may measure and/or mix the proper ratios of the different types of insulins to arrive at the determined strength. At block 614, logic flow 600 may deliver the insulin formulation to a user, for example, via insulin delivery device 102.

In an alternative embodiment, instead of formulating and/or delivering an insulin formulation, the insulin formulation process may provide the user with an alert or other message suggesting that the patient supplement their insulin, for instance, with a fast (or faster) acting insulin separately. The fast acting insulin delivery modes can be oral, inhaled, or injected. In some embodiments, the insulin formulation process may not generate or deliver an insulin formulation if there are not sufficient insulin types (for instance, rapid-acting insulin) to generate a determined formulation. In such embodiments in which there is not a sufficient insulin type, the insulin formulation process may generate an alert for the user. For example, other forms of insulins, such as approved inhaled insulin dosage, can be suggested by the insulin formulation process, for instance, upon detecting a meal or carbohydrate ingestion event.

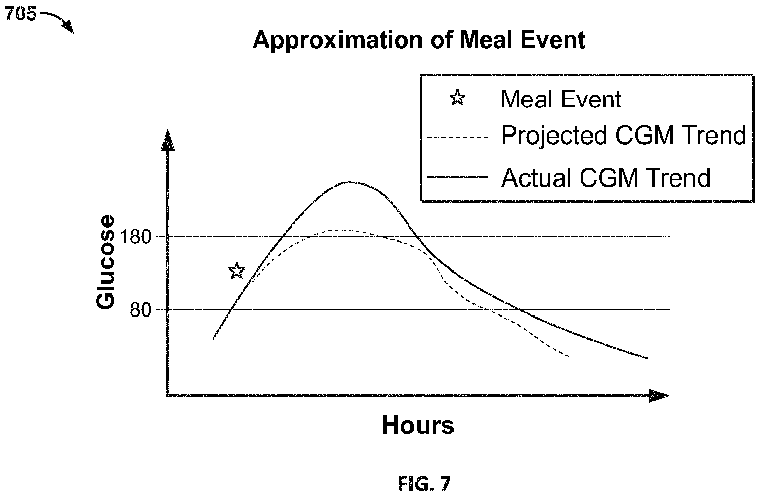

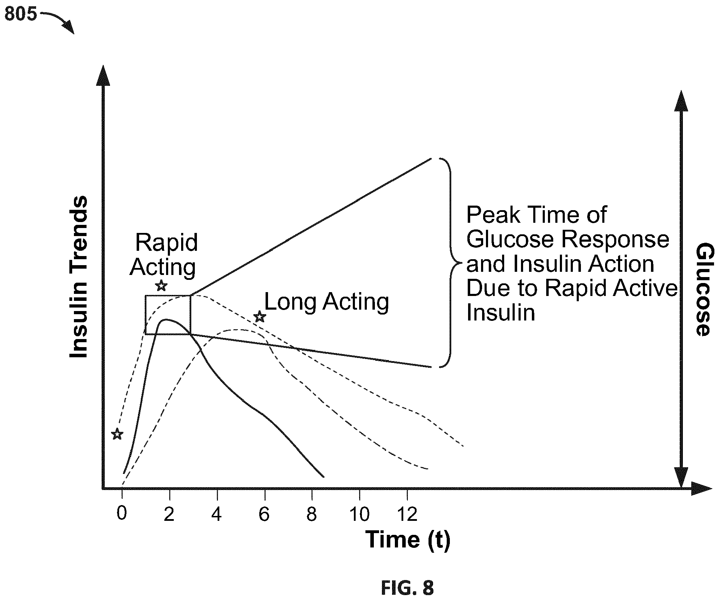

**FIG. 7** depicts an illustrative plot of an approximation of a meal event according to some embodiments. For example, graph 705 depicts a plot of a projected CGM trend and an actual CGM trend in view of a meal event over glucose versus time. As shown in FIG. 7, there is a deviation between the actual CGM trend and the projected CGM trend. **FIG. 8** depicts an illustrative plot of a peak time window to coincide insulin action to peak glucose response according to some embodiments. As shown in FIG. 8, a peak time of a glucose response for a rapid-acting insulin may be determined for a user.

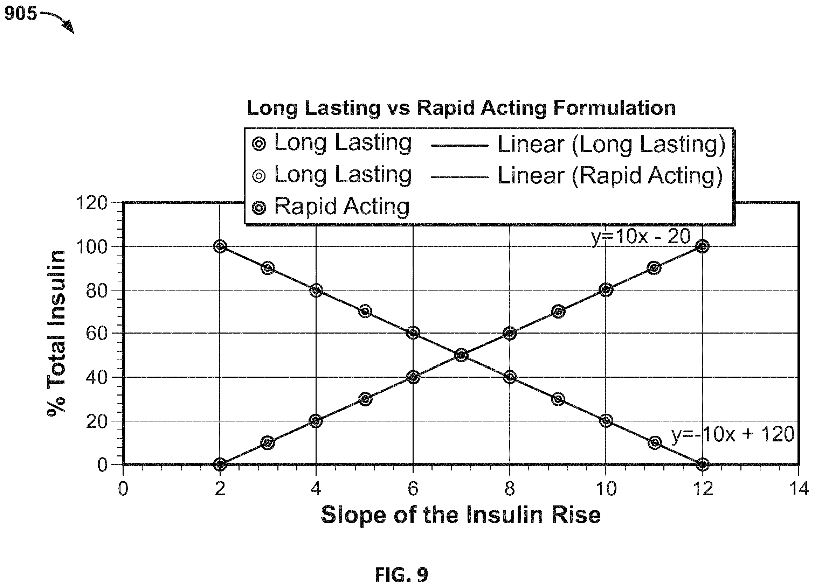

**FIG. 9** depicts an illustrative plot 905 comparing long lasting insulin versus rapid-acting insulin according to some embodiments. In various embodiments, a rapid acting and long lasting proportion may be calculated based upon the slope of the CGM rise. The blood glucose rise, for example, >= 12 mg/dL in a 5 minute interval, may indicate faster onset needed which may be supplied with 100% rapid acting insulin. Alternatively, a small rise, for example, a smaller slope of <= 2 mg/dL, may indicate that more long acting insulin is needed. A slope of 0 or a negative slope may indicate that no additional correction is needed and the insulin formulation process may suggest suspension.

The following Table 1 provides a mapping of yields that total 100% formulation for both long lasting and rapid acting insulin types. In some embodiments, the mapping provided in Table 1 may be used to derive a mathematical representation of an insulin formulation.

TABLE 1

| CGM Slope | Long Lasting | Rapid Acting | Total (%) |
|---|---|---|---|
| 2 | 100 | 0 | 100 |
| 3 | 90 | 10 | 100 |
| 4 | 80 | 20 | 100 |
| 5 | 70 | 30 | 100 |
| 6 | 60 | 40 | 100 |
| 7 | 50 | 50 | 100 |
| 8 | 40 | 60 | 100 |
| 9 | 30 | 70 | 100 |
| 10 | 20 | 80 | 100 |
| 11 | 10 | 90 | 100 |
| 12 | 0 | 100 | 100 |

The following Equation (2) may be used to calculate the required amount I(total) insulin, to correct CGM rise at time $t$:

$$I(total) = I(rapid\ acting) + I(long\ acting)$$

$$I(rapid\ acting) = 10 * x - 20$$

$$I(long\ acting) = -10 * x + 120,$$

where *x* is the slope of the CGM. In some embodiments, *x* may be determined by the following

Equation (3):

$$x = CGM(k) - CGM(k - 1),$$

where *k* represents the control cycle.

The insulin formulation process in conjunction with Equations (2) and (3) may operate in a feedback loop to deliver the right amount of rapid acting and/or long acting insulin as needed. For example, if the rise of insulin is faster but the rapid acting portion is not sufficient, then CGM rise may be higher for the next control cycle or two, which may in turn generate a higher insulin requirement from the insulin formulation process. This higher insulin need may translate to a higher-percentage formulation of rapid acting insulin and thus will have better ability to correct the CGM rise. Accordingly, a feedback mechanism may handle blood glucose rise due to various carbohydrate types (for example, slow soluble vs high sugar, high carb vs high fat, and/or the like) effectively.

In some embodiments, when there is a slow rise or a downward trend, the insulin formulation process may suggest either basal insulin or suspension. In various embodiments, the basal delivery may be in response to small increase in CGM and small CGM slope. Accordingly, the insulin formulation process may formulate higher doses of long acting insulin suitable for basal delivery.

While the present disclosure has been illustrated and described in detail in the drawings and foregoing description, the same is to be considered as illustrative and not restrictive in character, it being understood that only the certain embodiments have been shown and described and that all changes, alternatives, modifications and equivalents that come within the spirit of the disclosure are desired to be protected.

It should be understood that while the use of words such as preferable, preferably, preferred or more preferred utilized in the description above indicate that the feature so described may be more desirable, it nonetheless may not be necessary and embodiments lacking the same may be contemplated as within the scope of the present disclosure, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

**Claims**

1. An automated insulin delivery device, comprising:

    a wireless interface with a glucose sensor for providing blood glucose concentration readings of a user;
    a plurality of insulin reservoirs, each of the plurality of insulin reservoirs holding a different one of a plurality of insulin types, each of the plurality of insulin types having a different strength;
    a storage medium storing programming instructions and user glucose information, the user glucose information comprising the blood glucose concentration readings, predicted future blood glucose concentration readings, and user activity information;
    a processor for executing the programming instructions in the storage media to:

        determine an insulin strength based on the user glucose information,
        determine an insulin formulation ratio to generate an insulin formulation having the insulin strength using the plurality of insulin types;

    an insulin formulation device to generate the insulin formulation; and
    an insulin delivery component to deliver the insulin formulation to the user.

2. The automated insulin delivery device of claim 1, the plurality of reservoirs comprising two reservoirs, a first reservoir of the two reservoirs holding a rapid-acting insulin and a second reservoir of the two reservoirs holding a long-acting insulin.

3. The automated insulin delivery device of one of the preceding claims, the user glucose information comprising at least one of a meal event value, a carbohydrate ingestion value, or an increased activity value, and/or

    the user glucose information comprising a meal event value determined based on a glucose concentration trend deviation factor and one or more secondary factors, and/or
    the user glucose information comprising a carbohydrate ingestion event value determined based on a glucose concentration trend deviation factor and one or more secondary factors.

4. The automated insulin delivery device of claim 3, the one or more secondary factors comprising at least one of a time of day or historical mealtimes of the user.

5. The automated insulin delivery device of one of the preceding claims, wherein the processor executes the programming instructions to access insulin strength information and determine the insulin strength by comparing at least one event value determined based on the user glucose information to the insulin strength information.

6. The automated insulin delivery device of one of the preceding claims, the plurality of insulin comprising at least two of rapid-acting insulin, long-acting insulin, short-acting insulin, or intermediate acting insulin (NPH).

7. The automated insulin delivery device of one of the preceding claims, the glucose information comprising a plurality of event values, each of the plurality of event values being assigned a weight for determining the insulin strength.

8. The automated insulin delivery device of one of the preceding claims, at least one of the plurality of reservoirs configured to store the insulin formulation for delivery to the user.

9. A method performed by an Automated Insulin Delivery (AID) system, the method comprising:

    determining blood glucose concentration readings of a user;
    storing a plurality of insulin types in a plurality of insulin reservoirs, each of the plurality of insulin types having a different strength;
    determining an insulin strength based on user glucose information;
    determining an insulin formulation ratio to generate an insulin formulation having the insulin strength using the plurality of insulin types;
    generating the insulin formulation; and
    delivering the insulin formulation to the user.

10. The method of claim 9, the plurality of reservoirs comprising two reservoirs, a first reservoir of the two reservoirs holding a rapid-acting insulin and a second reservoir of the two reservoirs holding a long-acting insulin.

11. The method of claim 9 or 10, the glucose information comprising at least one of a meal event value, a carbohydrate ingestion value, or an increased activity value.

12. The method of one of claims 9 to 11, the glucose information comprising a meal event value determined based on a glucose concentration trend deviation factor and one or more secondary factors, and/or
the glucose information a carbohydrate ingestion event value determined based on a glucose concentration trend deviation factor and one or more secondary factors.

13. The method of claim 12, the one or more secondary factors comprising at least one of a time of day or historical mealtimes of the user.

14. The method of one of claims 9 to 13, comprising accessing insulin strength information and determining the insulin strength by comparing at least one event value determined based on the user glucose information to the insulin strength information,
the glucose information comprising a plurality of event values, each of the plurality of event values being assigned a weight for determining the insulin strength.

15. The method of one of claims 9 to 14, the plurality of insulin comprising at least two of rapid-acting insulin, long-acting insulin, short-acting insulin, or intermediate acting insulin (NPH).

Cloud Services/ Server(s) 128

Network 124

Management Device 104
Processor 119
UI 123
Control App. 120
Storage 118
Histories 121

100

Insulin Delivery Device 102
Controller 110
Insulin Reservoirs
112a ... 112n
Insulin Formulation Device 120
Histories 113
Storage 114
Control App. 116
User Interface 117

Glucose Monitor 106

User 108

Computing Device 126

Network 122

FIG. 1

200

FIG. 2

## 300

```
┌─────────────────────────────────────┐
│  Determine Blood Glucose Information  │ ◄─────────────┐
│                 302                   │               │
└─────────────────────────────────────┘               │
                    │                                   │
                    ▼                                   │
┌─────────────────────────────────────┐               │
│ Determine Predicted Future Blood     │               │
│ Glucose Concentration Value          │               │
│                 304                   │               │
└─────────────────────────────────────┘               │
                    │                                   │
                    ▼                                   │
┌─────────────────────────────────────┐               │
│   Determine Trend Deviation Factor    │               │
│                 306                   │               │
└─────────────────────────────────────┘               │
                    │                        ┌──────────────────────┐
                    ▼                        │  Next Cycle 316      │ ◄──┐
┌─────────────────────────────────────┐    └──────────────────────┘    │
│   Determine Current Insulin Dosage   │               ▲                │
│   Information                        │               │                │
│                 308                   │               │                │
└─────────────────────────────────────┘               │                │
                    │                                   │                │
                    ▼                                   │                │
┌─────────────────────────────────────┐               │                │
│    Determine Secondary Factors        │               │                │
│                 310                   │               │                │
└─────────────────────────────────────┘               │                │
                    │                                   │                │
                    ▼                          ┌──────────────────────┐ │
              ◇ Start of Meal? ◇ ──No──►      │  Reset Meal Event    │ │
                    312                        │  Value               │─┘
                    │                          │        314           │
                   Yes                         └──────────────────────┘
                    │
                    ▼
┌─────────────────────────────────────┐
│    Determine Meal Event Value         │────────────────────────────┘
│                 318                   │
└─────────────────────────────────────┘
```

**FIG. 3**

400

```
┌─────────────────────────────────────┐
│  Determine Blood Glucose Information  │ ◄──────────┐
│                402                    │            │
└─────────────────────────────────────┘            │
                  │                                  │
                  ▼                                  │
┌─────────────────────────────────────┐            │
│ Determine Predicted Future Blood Glucose │        │
│         Concentration Value          │            │
│                404                    │            │
└─────────────────────────────────────┘            │
                  │                                  │
                  ▼                          ┌──────────────┐
┌─────────────────────────────────────┐    │  Next Cycle  │
│   Determine Trend Deviation Factor   │    │     414      │ ◄──┐
│                406                    │    └──────────────┘    │
└─────────────────────────────────────┘            ▲           │
                  │                                  │           │
                  ▼                                  │           │
┌─────────────────────────────────────┐            │           │
│     Determine Secondary Factors      │    ┌────────────────┐ │
│                408                    │    │      Reset     │ │
└─────────────────────────────────────┘    │  Carbohydrate  │ │
                  │                          │Ingestion Event │ │
                  ▼                          │     Value      │ │
              ◇                              │      412       │ │
         Carbohydrate ──── No ─────────────►└────────────────┘ │
          Ingestion?                                            │
             410                                                │
              ◇                                                 │
              │ Yes                                             │
              ▼                                                 │
┌─────────────────────────────────────┐                       │
│ Determine Carbohydrate Ingestion Event │                     │
│              Value                    │─────────────────────┘
│                416                    │
└─────────────────────────────────────┘
```

**FIG. 4**

**500**

Determine Activity Information
502

Determine Hypoglycemia Mode
504

Determine Insulin Sensitivity Level
506

Determine Trend Deviation Factor
508

Determine Secondary Factors
510

Increased Activity?
512

Resent Elevated Activity Event Value
514

Next Cycle 516

Determine Elevated Activity Indicator Value
518

**FIG. 5**

**600**

```
┌─────────────────────────────────────┐
│      Determine Meal Event Value       │
│                 602                   │
└─────────────────────────────────────┘
                    │
                    ▼
┌─────────────────────────────────────┐
│  Determine Carbohydrate Ingestion Event│
│                Value                  │
│                 604                   │
└─────────────────────────────────────┘
                    │
                    ▼
┌─────────────────────────────────────┐
│   Determine Elevated Activity Indicator│
│                Value                  │
│                 606                   │
└─────────────────────────────────────┘
                    │
                    ▼
┌─────────────────────────────────────┐
│  Determine Insulin Strength Based on Event│
│                Values                 │
│                 608                   │
└─────────────────────────────────────┘
                    │
                    ▼
┌─────────────────────────────────────┐
│ Determine Formulation of Different Insulin│
│   Types to Achieve Insulin Strength   │
│                 610                   │
└─────────────────────────────────────┘
                    │
                    ▼
┌─────────────────────────────────────┐
│     Generate Insulin Formulation      │
│                 612                   │
└─────────────────────────────────────┘
                    │
                    ▼
┌─────────────────────────────────────┐
│      Deliver Insulin Formulation      │
│                 614                   │
└─────────────────────────────────────┘
```

**FIG. 6**

FIG. 7

EP 3 960 219 A1

FIG. 8

FIG. 9

EP 3 960 219 A1

Europäisches
Patentamt

European
Patent Office

Office européen
des brevets

# EUROPEAN SEARCH REPORT

Application Number

EP 21 19 3526

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/101225 A1 (O'CONNOR JASON [US] ET AL) 2 April 2020 (2020-04-02) * paragraph [0014] – paragraph [0115]; figures 1,2,5 * | 1-15 | INV. A61M5/14 A61M5/142 A61M5/172 G16H20/17 |
| A | WO 2009/134380 A2 (HALOZYME INC [US]; FROST GREGORY I [US] ET AL.) 5 November 2009 (2009-11-05) * claims 68,69 * | 1-15 | |
| A | WO 2018/136799 A1 (MEDTRONIC MINIMED INC [US]) 26 July 2018 (2018-07-26) * page 47, line 21 – page 48, line 6; figure 1 * | 1-15 | |

TECHNICAL FIELDS
SEARCHED       (IPC)

A61M
G16H
A61K

The present search report has been drawn up for all claims

| Place of search | Date of completion of the search | Examiner |
|---|---|---|
| Munich | 24 January 2022 | Feber, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 3526

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2022

| Patent document cited in search report | Publication date | Patent family member(s) | | Publication date |
|---|---|---|---|---|
| US 2020101225 A1 | 02-04-2020 | AU | 2019347755 A1 | 13-05-2021 |
| | | CA | 3112209 A1 | 02-04-2020 |
| | | CN | 112789070 A | 11-05-2021 |
| | | EP | 3856285 A1 | 04-08-2021 |
| | | JP | 2022501139 A | 06-01-2022 |
| | | US | 2020101225 A1 | 02-04-2020 |
| | | WO | 2020069406 A1 | 02-04-2020 |
| WO 2009134380 A2 | 05-11-2009 | AR | 072250 A1 | 18-08-2010 |
| | | AU | 2009241795 A1 | 05-11-2009 |
| | | BR | PI0911782 A2 | 13-03-2018 |
| | | CA | 2722628 A1 | 05-11-2009 |
| | | CL | 2009001014 A1 | 19-06-2009 |
| | | CN | 102083458 A | 01-06-2011 |
| | | CY | 1116311 T1 | 08-02-2017 |
| | | DK | 2300046 T3 | 23-03-2015 |
| | | EA | 201001698 A1 | 30-08-2011 |
| | | EP | 2300046 A2 | 30-03-2011 |
| | | EP | 2705850 A2 | 12-03-2014 |
| | | ES | 2531630 T3 | 18-03-2015 |
| | | ES | 2572267 T3 | 31-05-2016 |
| | | HK | 1150967 A1 | 20-01-2012 |
| | | HK | 1195728 A1 | 21-11-2014 |
| | | HR | P20150313 T1 | 05-06-2015 |
| | | JP | 5809330 B2 | 10-11-2015 |
| | | JP | 2011523940 A | 25-08-2011 |
| | | JP | 2014208686 A | 06-11-2014 |
| | | JP | 2016034959 A | 17-03-2016 |
| | | KR | 20110021818 A | 04-03-2011 |
| | | KR | 20140093759 A | 28-07-2014 |
| | | MX | 348276 B | 02-06-2017 |
| | | NZ | 588748 A | 28-09-2012 |
| | | PE | 20091975 A1 | 09-01-2010 |
| | | PE | 20141384 A1 | 10-10-2014 |
| | | PL | 2300046 T3 | 30-06-2015 |
| | | PT | 2300046 E | 08-04-2015 |
| | | SG | 10201404218V A | 30-10-2014 |
| | | SI | 2300046 T1 | 30-04-2015 |
| | | TW | 201000121 A | 01-01-2010 |
| | | US | 2009304665 A1 | 10-12-2009 |
| | | US | 2012251517 A1 | 04-10-2012 |
| | | US | 2014135682 A1 | 15-05-2014 |
| | | UY | 31789 A1 | 31-08-2009 |
| | | WO | 2009134380 A2 | 05-11-2009 |
| | | ZA | 201007403 B | 26-06-2019 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 19 3526

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2022

| Patent document cited in search report | Publication date | Patent family member(s) | Publication date |
|---|---|---|---|
| WO 2018136799 A1 | 26-07-2018 | CA 3049779 A1 | 26-07-2018 |
| | | CN 110461407 A | 15-11-2019 |
| | | EP 3570932 A1 | 27-11-2019 |
| | | US 2018200412 A1 | 19-07-2018 |
| | | WO 2018136799 A1 | 26-07-2018 |

EPO FORM P0459

For more details about this annex : see Official Journal of the European Patent Office, No. 12/82

page 2 of 2